# EUROPEAN PATENT APPLICATION

(11) **EP 3 943 016 A1**
(43) Date of publication of application: **26.01.2022**
(21) Application number: 21186599.3
(22) Date of filing: 20.07.2021
(51) Int. Cl.: A61B 17/068, A61B 17/064

(54) **CARTRIDGE ALIGNMENT MECHANISM FOR USE WITH SURGICAL DEVICES**

(30) Priority: 21.07.2020 US 202063054438 P; 25.05.2021 US 202117329694
(71) Applicant: Covidien LP, Mansfield, MA 02048 (US)
(72) Inventor: SNIFFIN, Kevin S., Roxbury, Connecticut 06783 (US); LAIRD, Brian S., Granby, Connecticut 06035 (US); FISCHVOGT, Gregory W., Reno, Nevada 89521 (US); CUTLER, Rebecca H., Wallingford, Connecticut 06492 (US); PRIBANIC, Russell V., Roxbury, Connecticut 06783 (US)
(74) Representative: Maschio, Antonio

(57) **Abstract**

A surgical device includes a cartridge alignment mechanism that loads a cartridge onto an engagement portion without having to place the cartridge in proper orientation with the engagement portion.

## Description

### CROSS-REFERENCE TO RELATED APPLICATION

This application claims the benefit of and priority to U.S. Provisional Patent Application Serial No. 63/054,438, filed July 21, 2020, the disclosure of which is incorporated by reference herein in its entirety.

### TECHNICAL FIELD

This disclosure relates to surgical devices for performing endoscopic surgical procedures. More specifically, this disclosure relates to a cartridge alignment mechanism for use with the surgical devices.

### BACKGROUND

Hernias are abnormal protrusions of an organ or other body structure through a defect or natural opening in a covering membrane, abdominal wall, muscle or bone. During surgical hernia repair procedures, surgical mesh materials are placed over the defect in the abdominal wall and are often sutured within the surgical opening by conventional suturing techniques. Traditionally, hernia repairs involved major invasive surgical procedures which often caused excessive trauma to the patient and necessitated long postoperative recuperative periods.

Recently, the use of laparoscopic and endoscopic surgical procedures has been relatively popular. During laparoscopic or endoscopic surgical procedures, access to a surgical site is achieved through a small incision or through a narrow cannula inserted through a small entrance wound in a patient.

### SUMMARY

In accordance with this disclosure, a surgical tack applier includes a reloadable cartridge supporting a plurality of tacks, a shipping clip including an outer body and an inner body rotatably supported within the outer body, and a tack retaining and advancing assembly configured to selectively release or fire the plurality of tacks. The reloadable cartridge includes a cartridge orientation guide having an axial protrusion extending radially inwards along a length of the reloadable cartridge. The reloadable cartridge is detachably secured with the inner body for concomitant rotation with the inner body. The tack retaining and advancing assembly includes an engagement portion detachably supporting the reloadable cartridge. The engagement portion includes an orientation member defining an orientation slot slidably receiving the axial protrusion of the cartridge orientation guide of the reloadable cartridge, and a camming surface that is tapered along a length of the engagement portion such that when the cartridge orientation guide of the reloadable cartridge is pushed against the camming surface, the reloadable cartridge rotates to align the axial protrusion of the cartridge orientation guide with the orientation slot of the orientation member of the engagement portion.

In an aspect, the inner body may include a flexible finger having a protrusion. The flexible finger may be radially deflectable.

In another aspect, the reloadable cartridge may define a bore dimensioned to receive the protrusion of the flexible finger of the inner body.

In another aspect, the protrusion may have a tapered surface that slides out of the bore of the reloadable cartridge when the reloadable cartridge and the inner body are axially moved apart.

In yet another aspect, the tack retaining and advancing assembly may include a drive shaft.

In another aspect, the drive shaft may be rotatable to selectively release or fire the plurality of tacks from the reloadable cartridge, and axially slidable between a first position and a second position.

In accordance with another aspect of the disclosure, a surgical tack applier includes a reloadable cartridge supporting a plurality of tacks, a shipping clip including an outer body and an inner body rotatably supported within the outer body, and a tack retaining and advancing assembly configured to selectively release or fire the plurality of tacks. The reloadable cartridge includes a cartridge orientation guide. The reloadable cartridge is detachably secured with the inner body for concomitant rotation with the inner body. The tack retaining and advancing assembly includes an engagement portion detachably supporting the reloadable cartridge. The engagement portion includes an orientation member operatively engaging the cartridge orientation guide of the reloadable cartridge and a camming surface that is tapered such that when the cartridge orientation guide of the reloadable cartridge is pushed against the camming surface. The reloadable cartridge rotates to align the cartridge orientation guide with the orientation member of the engagement portion.

In an aspect, the reloadable cartridge may include a cartridge locking portion that is biased towards a lumen defined by the reloadable cartridge.

In another aspect, the cartridge locking portion may define an acute angle with respect to a longitudinal axis defined by the reloadable cartridge.

In still yet another aspect, the engagement portion may define a recess and include a finger disposed within the recess. The finger may selectively engage the cartridge locking portion to selectively secure the reloadable cartridge with the engagement portion.

In an aspect, the cartridge locking portion of the reloadable cartridge may be transitionable between a secured position, in which, the cartridge locking portion is disposed within the recess of the engagement portion, and a releasable position, in which, the cartridge locking portion is disposed radially outwards of the recess.

In an aspect, the tack retaining and advancing assembly may include a drive shaft. The drive shaft may be rotatable to selectively release or fire the plurality of tacks from the reloadable cartridge, and axially slidable between a first position and a second position. When the drive shaft is in the first position, the cartridge locking portion of the reloadable cartridge may be disposed within the recess such that the reloadable cartridge is securely coupled to the engagement portion, and when the drive shaft is in the second position, the finger of the engagement portion may urge the cartridge locking portion of the reloadable cartridge out of the recess such that the reloadable cartridge is releasable from the engagement portion.

In accordance with another aspect of the disclosure, a cartridge alignment mechanism for use with a surgical device includes a cartridge supporting a plurality of anchors, a shipping clip including an outer body and an inner body rotatably supported within the outer body, and an engagement portion detachably supporting the cartridge. The cartridge includes a cartridge orientation guide. The cartridge is removably coupled to the inner body for concomitant rotation with the inner body. The engagement portion includes an orientation member slidably engaging the cartridge orientation guide of the cartridge and a camming surface leading into the orientation member such that when the camming surface is pushed against the cartridge orientation guide of the reloadable cartridge, the cartridge orientation guide of the cartridge aligns with and engages the orientation member of the engagement portion.

### BRIEF DESCRIPTION OF DRAWINGS

The above and other aspects and features of this disclosure will become more apparent in view of the following detailed description when taken in conjunction with the accompanying drawings wherein like reference numerals identify similar or identical elements.
FIG. 1 is a perspective view of an endoscopic surgical tack applier in accordance with this disclosure, illustrating a shipping clip supporting a reloadable cartridge separated from an endoscopic tack retaining/advancing assembly of the endoscopic surgical tack applier;
FIG. 2 is an enlarged perspective view of the indicted area of detail of FIG. 1;
FIG. 3 is an enlarged perspective view of the indicated area of detail of FIG. 2;
FIG. 4 is an exploded perspective view of the shipping clip of FIG. 1 with parts separated;
FIG. 4A is a perspective view of the shipping clip of FIG. 1;
FIG. 4B is a side view of the shipping clip of FIG. 4A;
FIG. 4C is a front end view of the shipping clip of FIG. 4A;
FIG. 4D is a top view of the shipping clip of FIG. 4A;
FIG. 4E is a rear end view of the shipping clip of FIG. 4A;
FIG. 4F is a side cross-sectional view of the shipping clip of FIG. 4A without the reloadable cartridge;
FIG. 4G is a side cross-sectional view of the reloadable cartridge of FIG. 4;
FIG. 5 is a perspective view of an inner body of the shipping clip of FIG. 4 taken along section line 5-5 of FIG. 4;
FIG. 6 is an enlarged perspective view of the indicated area of detail of FIG. 4;
FIG. 7 is a perspective view of the reloadable cartridge of FIG. 4 taken along section line 7-7 of FIG. 4;
FIG. 8 is a perspective view of a tack of the reloadable cartridge of FIG. 7;
FIG. 9 is a cross-sectional view of the shipping clip supporting the reloadable cartridge of FIG. 4 taken along section line 9-9 of FIG. 2;
FIG. 10 is an enlarged view of the indicated area of detail of FIG. 9;
FIG. 11 is a cross-sectional view of the shipping clip of FIG. 9 taken along section line 11-11 of FIG. 9;
FIG. 12 is an enlarged view of the indicated area of detail of FIG. 11;
FIG. 13 is a partial cross-sectional view of the endoscopic tack retaining/advancing assembly of FIG. 2 taken along section line 13-13 of FIG. 2;
FIG. 14 is a partial perspective view of the endoscopic tack retaining/advancing assembly of FIG. 2 illustrating engagement with the shipping clip of FIG. 9 shown in phantom;
FIG. 15 is a cross-sectional view of the endoscopic tack retaining/advancing assembly and the shipping clip of FIG. 14 taken along section line 15-15 of FIG. 14;
FIG. 16 is a partial perspective view of the endoscopic tack retaining/advancing assembly of FIG. 3 illustrating coupling of the endoscopic tack retaining/advancing assembly of FIG. 2 with the shipping clip shown in phantom;
FIG. 17 is a cross-sectional view of the endoscopic tack retaining/advancing assembly and the shipping clip of FIG. 16 taken along section line 17-17 of FIG. 16;
FIG. 18 is a partial perspective view of the endoscopic tack retaining/advancing assembly of FIG. 2, illustrating the shipping clip aligned with the engagement portion of the endoscopic tack retaining/advancing assembly;
FIG. 19 is a cross-sectional view of the endoscopic tack retaining/advancing assembly and the shipping clip of FIG. 18 taken along section line 19-19 of FIG. 18;
FIG. 20 is a partial cross-sectional view of the shipping clip coupled to the endoscopic tack retaining/advancing assembly taken along section line 20-20 of FIG. 19;
FIG. 21 is a partial cross-sectional view of the shipping clip and the endoscopic tack retaining/advancing assembly, illustrating detachment of the shipping clip from the reloadable cartridge secured to the endoscopic tack retaining/advancing assembly;
FIG. 22 is an enlarged view of the indicated area of detail of FIG. 21;
FIG. 23 is a perspective view of the reloadable cartridge secured to the endoscopic tack retaining/advancing assembly; and
FIG. 24 is a partial cross-sectional view of the reloadable cartridge and the endoscopic tack retaining/advancing assembly of FIG. 23, illustrating detachment of the reloadable cartridge from the endoscopic tack retaining/advancing assembly.

### DETAILED DESCRIPTION

The endoscopic surgical device disclosed herein is described in detail with reference to the drawings, in which like reference numerals designate identical or corresponding elements in each of the several views. As used herein, the term "distal" refers to the portion that is being described which is farther from a user, while the term "proximal" refers to the portion that is being described which is closer to a user. In addition, the terms parallel and perpendicular are understood to include relative configurations that are substantially parallel and substantially perpendicular up to about + or - 10 degrees from true parallel and true perpendicular. Further, to the extent consistent, any or all of the aspects detailed herein may be used in conjunction with any or all of the other aspects detailed herein.

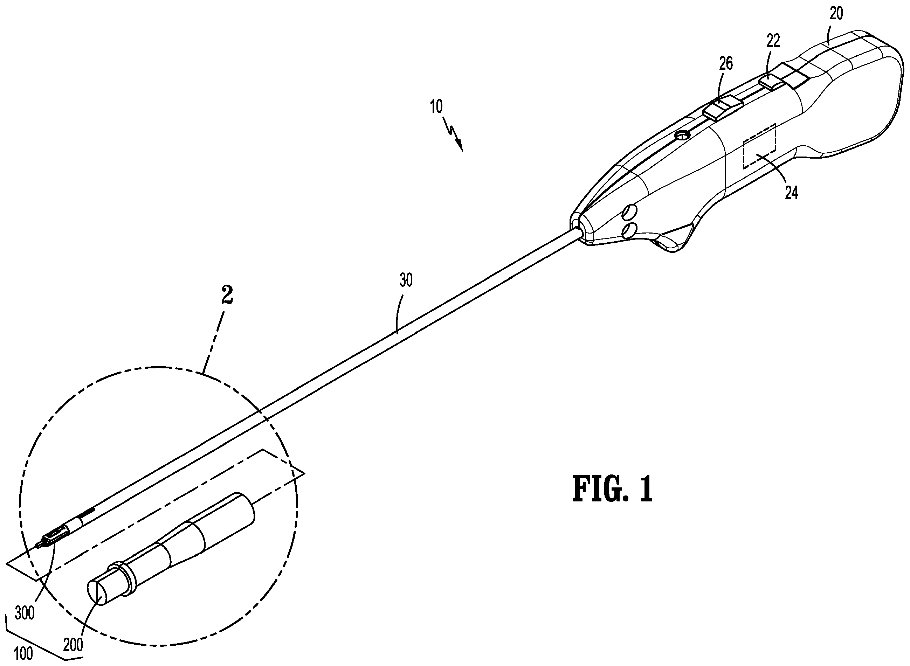

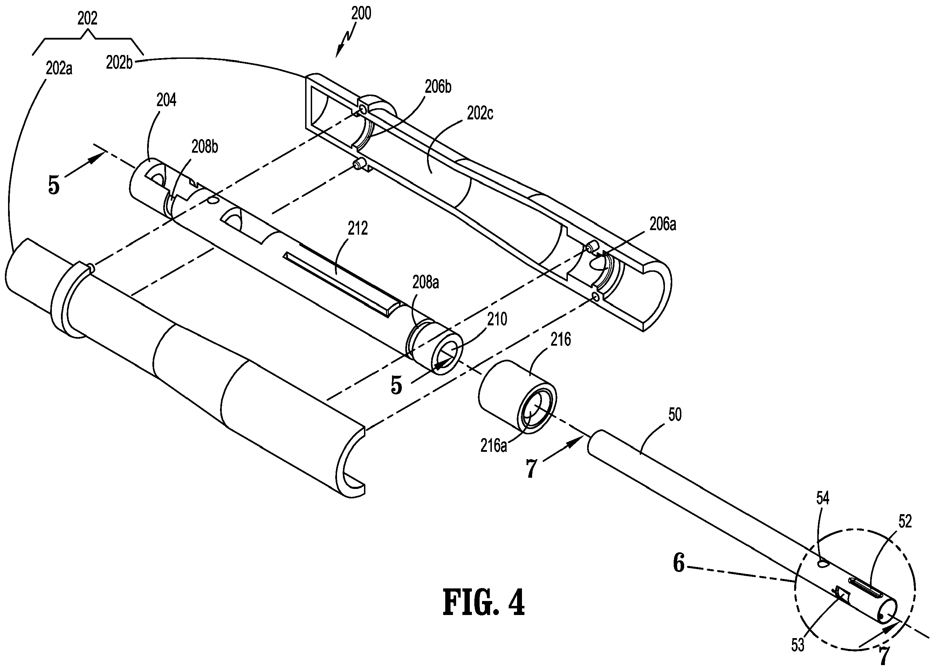

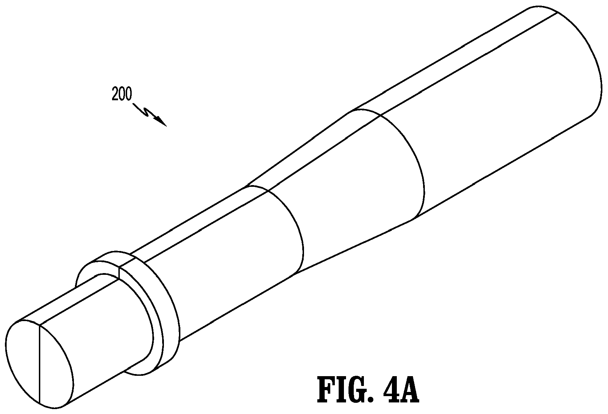

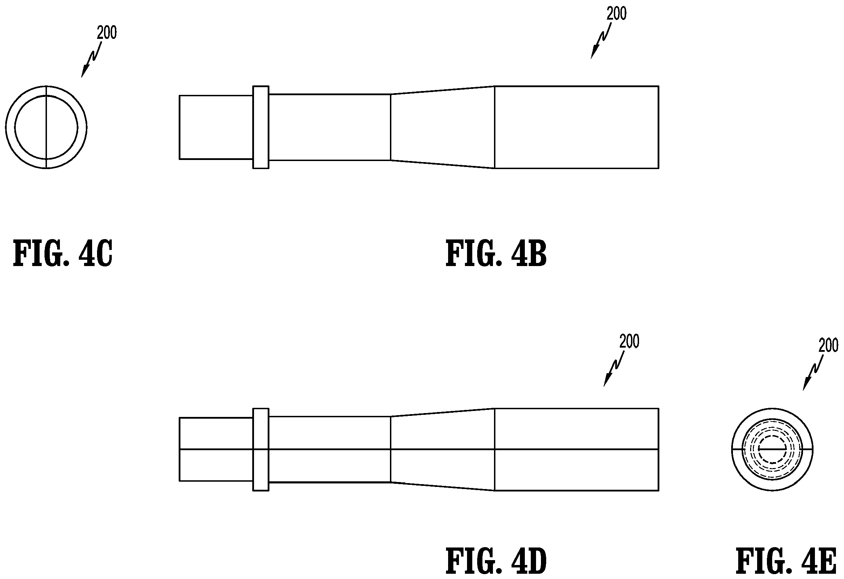

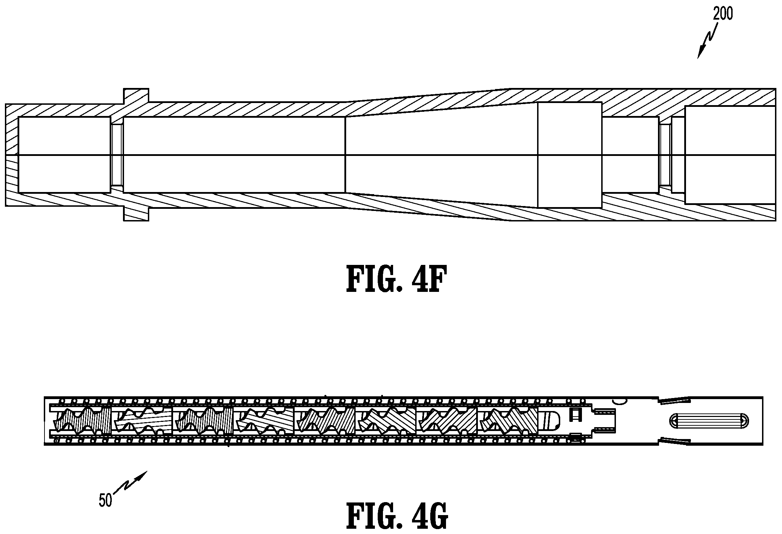

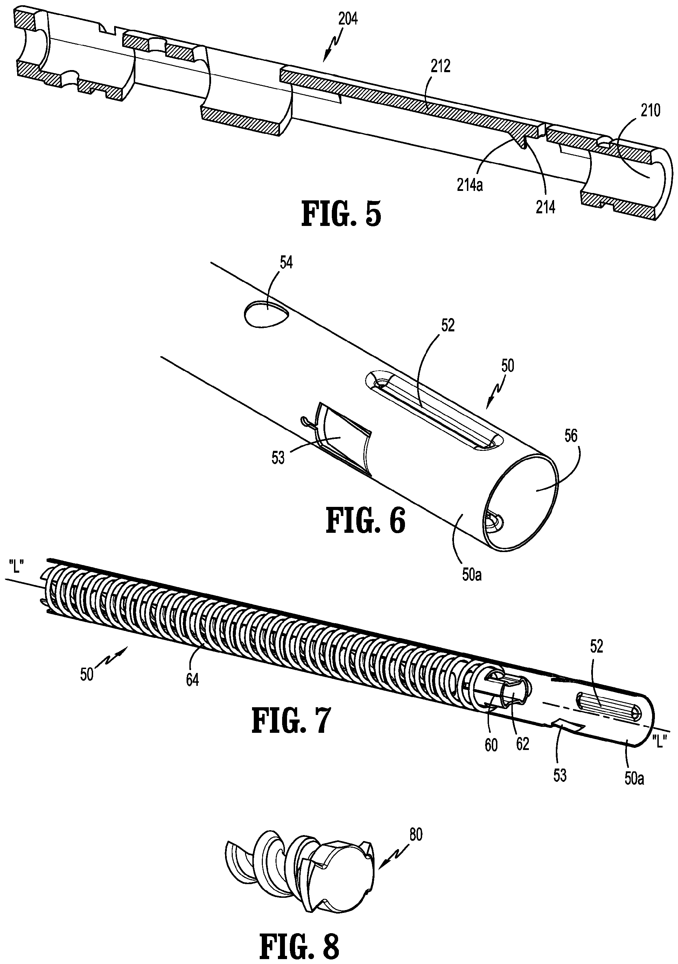

In FIG. 1, an exemplary cartridge alignment mechanism for use with an endoscopic surgical device, in the form of an endoscopic surgical tack applier 10 is shown generally as 100. The endoscopic surgical tack applier 10 includes a handle assembly 20 and an endoscopic tack retaining/advancing assembly 30 extending from the handle assembly 20 and configured to selectively release or fire a plurality of tacks or anchors 80 (FIG. 8). An engagement portion 300 of the endoscopic tack retaining/advancing assembly 30 detachably supports a reloadable cartridge 50 (FIG. 4) including the plurality of tacks 80. The cartridge alignment mechanism 100 includes a shipping clip 200 (see also, e.g., FIGS. 4A-4F) detachably supporting the reloadable cartridge 50 (see also, e.g., FIG. 4G), and the engagement portion 300 of the endoscopic tack retaining/advancing assembly 30 configured to operatively engage the reloadable cartridge 50 supported in the shipping clip 200. The cartridge alignment mechanism 100 facilitates loading of the reloadable cartridge 50 onto the endoscopic tack retaining/advancing assembly 30. Specifically, the cartridge alignment mechanism 100 aligns the reloadable cartridge 50 to the engagement portion 300 without having to place the reloadable cartridge 50 in proper orientation (e.g., radial orientation) with the engagement portion 300, as will be discussed hereinbelow.

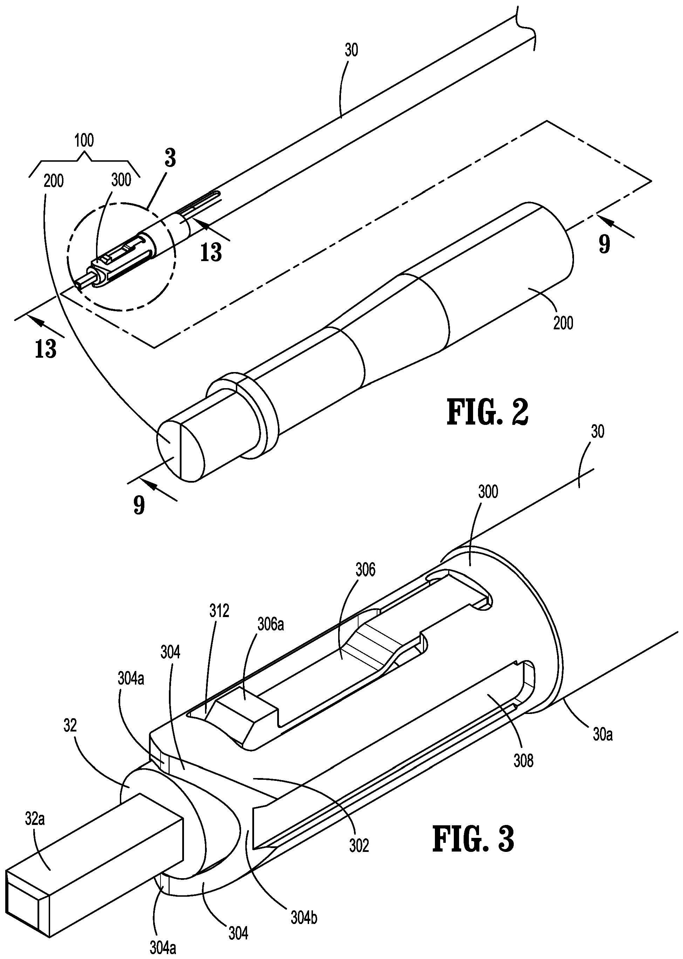

The handle assembly 20 includes a button 22 configured to fire the plurality of tacks 80 (FIG. 8) from the reloadable cartridge 50 (FIG. 4) mounted on the engagement portion 300 of the endoscopic tack retaining/advancing assembly 30. In particular, the button 22 is operatively connected to a drive mechanism as known by one skilled in the art such that actuation of the button 22 results in rotation of a drive shaft 32 (FIG. 3) of the tack retaining/advancing assembly 30 which, in turn, selectively fires the tacks 80 from the reloadable cartridge 50 through, e.g., a surgical mesh, and into tissue. For example, the drive mechanism may include, e.g., a flexible shaft, to impart rotation to the reloadable cartridge 50. In particular, the drive mechanism may be coupled to an actuator 24 such as, e.g., an electric motor, in the handle assembly 20 such that actuation of the button 22 results in rotation of the drive shaft 32. However, the drive mechanism may be mechanically actuated. In addition, the handle assembly 20 further includes a slider 26 that is operatively coupled to the drive shaft 32 to impart axial displacement to the drive shaft 32. The slider 26 may be mechanically or electro-mechanically actuated as known by one skilled in the art.

FIG. 2 illustrates the cartridge alignment mechanism 100 including the shipping clip 200 and the engagement portion 300 of the endoscopic tack retaining and advancing assembly 30. FIG. 3 illustrates the engagement portion 300 configured to operatively engage the reloadable cartridge 50 disposed within the shipping clip 200 (FIG. 2). The engagement portion 300 is at a distal end portion 30a of the endoscopic tack retaining/advancing assembly 30. The endoscopic tack retaining/advancing assembly 30 defines a channel extending through the engagement portion 300. The channel is configured to receive the drive shaft 32 therein. The drive shaft 32 includes a mating tip 32a extending distally from the engagement portion 300. The mating tip 32a is configured to engage a tack holder 60 (FIG. 7) of the reloadable cartridge 50, as will be discussed. The mating tip 32a may have a non-circular cross-section to facilitate concomitant rotation with the tack holder 60. The engagement portion 300 includes a distal end portion 302 having a camming surface 304 configured to engage cartridge orientation guides 52 (FIG. 4) of the reloadable cartridge 50, as will be discussed. The camming surface 304 may be tapered along a length of the engagement portion 300. The engagement portion 300 defines a recess 312. The engagement portion 300 further includes a finger 306 disposed in the recess 312. The finger 306 is radially flexible. The finger 306 includes a release member 306a configured to selectively engage a cartridge locking portion 53 (FIG. 6) of the reloadable cartridge 50, as will be discussed hereinbelow. The engagement portion 300 further defines orientation slots 308 (only one shown) dimensioned to slidably receive the respective cartridge orientation guides 52 (FIG. 4) of the reloadable cartridge 50. The orientation slots 308 may diametrically oppose each other. The orientation slots 308 extend from a proximal-most portion 304b of the camming surface 304 such that when the cartridge orientation guides 52 rotatably engage the camming surface 304, the cartridge orientation guides 52 are directed into the respective orientation slots 308 of the engagement portion 300. For example, the camming surface 304 may include a pair of diametrically opposing distal-most portions 304a and a pair of diametrically opposing proximal-most portions 304b (only one shown). Each orientation slot 308 extends axially to slidably receive the corresponding cartridge orientation guide 52.

FIGS. 4 and 5 illustrate the shipping clip 200 including an outer body portion 202 and an inner body portion 204. The outer body portion 202 includes first and second body halves 202a, 202b that define a cavity 202c rotatably supporting the inner body portion 204 therein. The outer body portion 202 includes proximal and distal ribs 206a, 206b extending radially inward. The inner body portion 204 includes proximal and distal circular grooves 208a, 208b configured to rotatably receive the respective proximal and distal ribs 206a, 206b of the outer body portion 202. The inner body portion 204 defines a lumen 210 therethrough. The lumen 210 is dimensioned to receive at least a portion of the reloadable cartridge 50. The inner body portion 204 further includes a finger 212 that is radially flexible. For example, the finger 212 may be radially biased inwards. The finger 212 includes a protrusion 214 extending radially inwards into the lumen 210. The protrusion 214 is configured to be received in a bore 54 (FIG. 6) of the reloadable cartridge 50 to secure the reloadable cartridge 50 with the inner body portion 204 for concomitant rotation therewith. The protrusion 214 includes a ramp 214a that enables the protrusion 214 to be disengaged from the bore 54 when the reloadable cartridge 50 is axially displaced from the shipping clip 200. The outer body portion 202 may include a non-uniform diameter. For example, a diameter of a proximal portion of the outer body portion 202 may be greater than a diameter of a distal portion of the outer body portion 202. The inner body portion 204 may further include a sleeve 216 (FIG. 4) defining a passage 216a therethrough. The sleeve 216 may be coupled to a proximal portion of the inner body portion 204 to be received in a proximal portion of the cavity 202c of the outer body portion 202. The passage 216a of the sleeve 216 is dimensioned to receive the reloadable cartridge 50 therethrough. It is contemplated that the sleeve 216 may be integrally formed with the inner body portion 204.

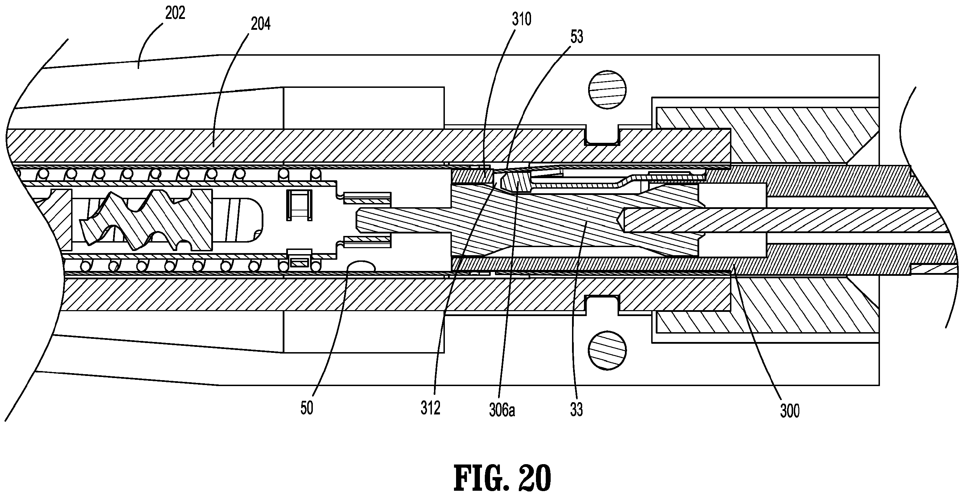

FIGS. 6-8 illustrate the reloadable cartridge 50 defining a lumen 56 therethrough. The lumen 56 is dimensioned to receive the tack holder 60 supporting serially arranged tacks 80. The tack holder 60 has a mouth 62 at a proximal portion of the tack holder 60 to receive the mating tip 32a (FIG. 3) of the drive shaft 32. The mouth 62 has a cross-section complementary to the cross-section of the mating tip 32a such that rotation of the drive shaft 32 imparts concomitant rotation to the tack holder 60. The reloadable cartridge 50 further includes a coil 64 disposed about the tack holder 60 such that rotation of the tack holder 60 advances the tacks 80 out of the reloadable cartridge 50. A proximal portion 50a of the reloadable cartridge 50 includes the cartridge orientation guides 52 that diametrically oppose each other. Each cartridge orientation guide 52 extends axially along a length of the reloadable cartridge 50. In addition, the proximal portion 50a further includes the cartridge locking portion 53 that secures the reloadable cartridge 50 to the engagement portion 300 (see FIG. 20) of the endoscopic tack retaining/advancing assembly 30, as will be discussed hereinbelow. The cartridge locking portion 53 may define an acute angle with respect to a longitudinal axis "L-L" defined by the reloadable cartridge 50. The cartridge locking portions 53 may diametrically oppose each other and may define an axis orthogonal to an axis defined by the cartridge orientation guides 52. The proximal portion 50a further defines the bore 54 distal of the cartridge orientation guides 52. The bore 54 is dimensioned to receive the protrusion 214 (FIG. 5) of the finger 212 of the inner body portion 204 to secure the reloadable cartridge 50 to the inner body portion 204 for concomitant rotation therewith.

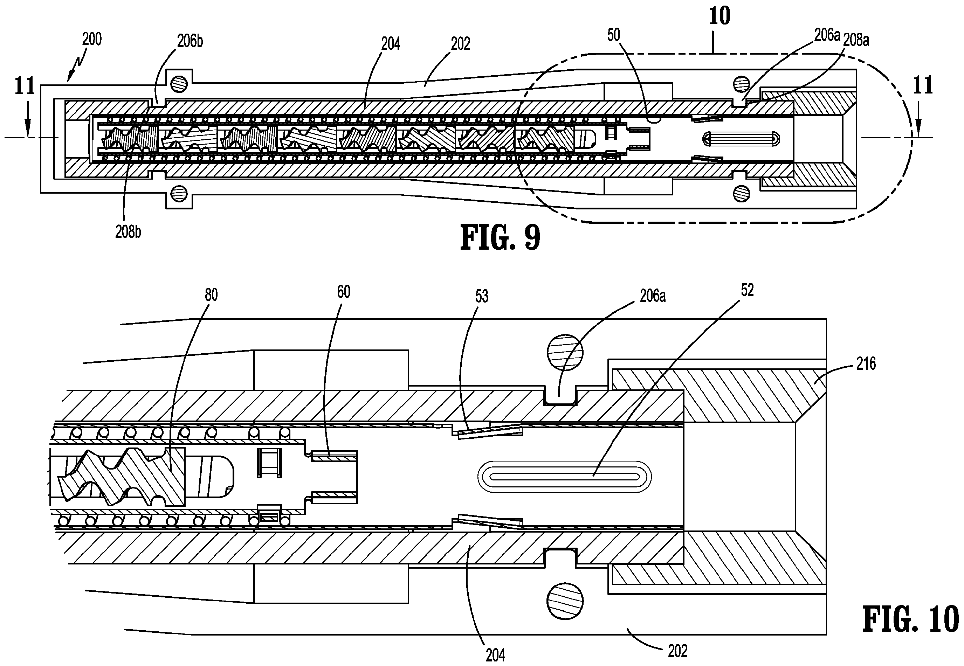

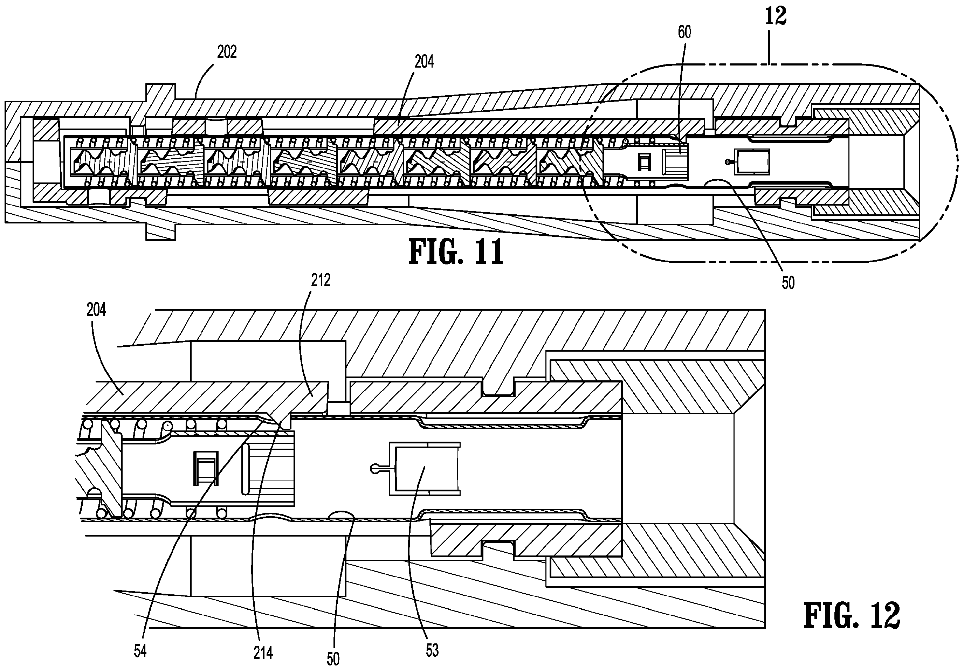

FIGS. 9 and 10 illustrate the reloadable cartridge 50 detachably supported within the inner body portion 204 of the shipping clip 200. The proximal and distal ribs 206a, 206b of the outer body portion 202 are received in the respective proximal and distal circular grooves 208a, 208b of the inner body portion 204 to enable rotation of the inner body portion 204 within the outer body portion 202 independent of the outer body portion 202. FIGS. 11 and 12 illustrate the reloadable cartridge 50 coupled to the inner body portion 204 to enable concomitant rotation. Specifically, the protrusion 214 that extends radially inward from the finger 212 of the inner body portion 204 is received in the bore 54 of the reloadable cartridge 50 to enable concomitant rotation.

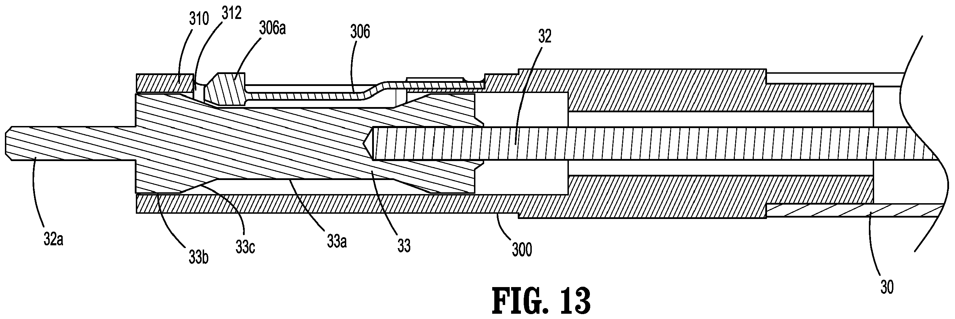

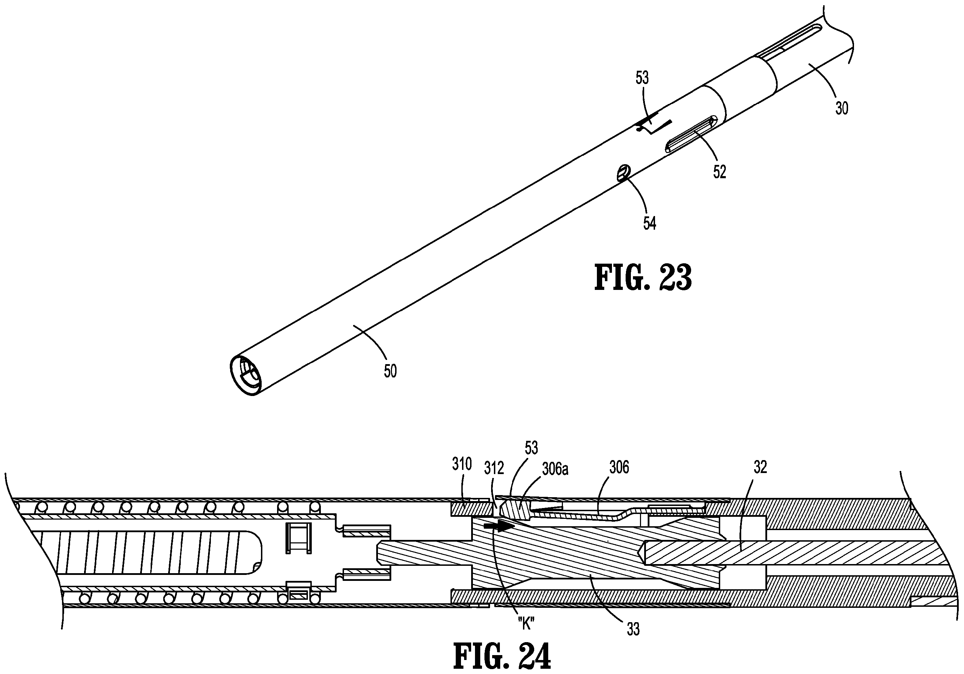

FIG. 13 illustrates the engagement portion 300 of the endoscopic tack retaining/advancing assembly 30. In particular, the engagement portion 300 includes the finger 306 disposed within the recess 312 defined in the engagement portion 300. The finger 306 has the release member 306a selectively securing the reloadable cartridge 50 (FIG. 4) to the engagement portion 300. The finger 306 is biased radially inwards, but radially flexible to enable outward displacement of the release member 306 away from the drive shaft 32. The drive shaft 32 has a head portion 33 rotatably and slidably supported in the engagement portion 300. In particular, the head portion 33 includes a first portion 33a having a first diameter and a second portion 33b distal of the first portion 33a and having a second diameter greater than the first diameter. The head portion 33 may further include a transition portion 33c that extends between the first and second portions 33a, 33b. When the head portion 33 is in a distal-most position within the engagement portion 300, the release member 306a of the finger 306 is disposed on the first portion 33a of the head portion 33. However, when the head portion 33 is retracted proximally, the release member 306 rides along the third portion 33c towards the second portion 33b, thereby extending the release member 306a radially outwards. Under such a configuration, the outward displacement of the release member 306a may transition the cartridge locking portion 53 (FIG. 6) of the reloadable cartridge 50 from a secured position (FIG. 20) to a released position (FIG. 24) in order to selectively secure the reloadable cartridge 50 to the engagement portion 300 of the endoscopic tack retaining/advancing assembly 30, as will be discussed hereinbelow.

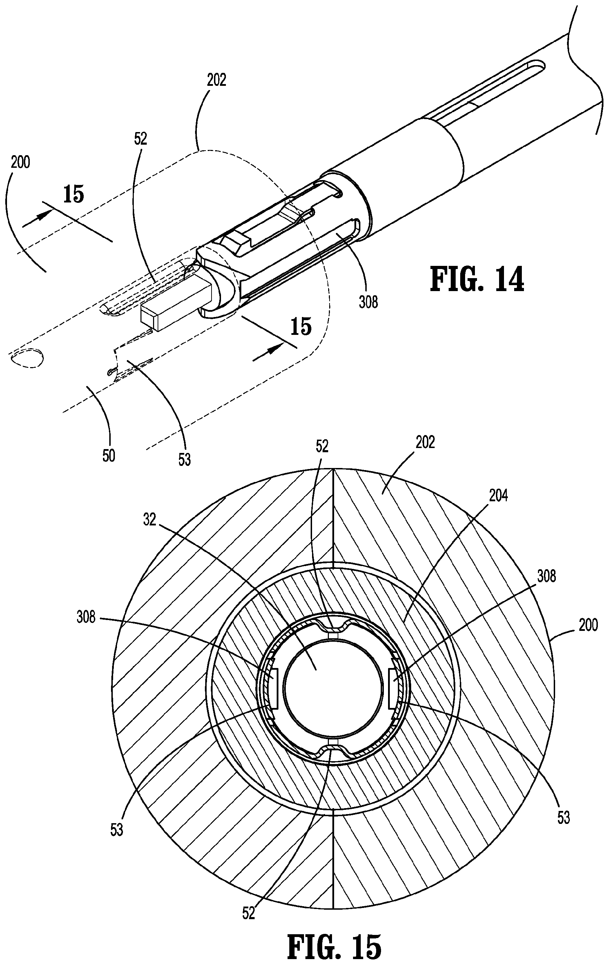

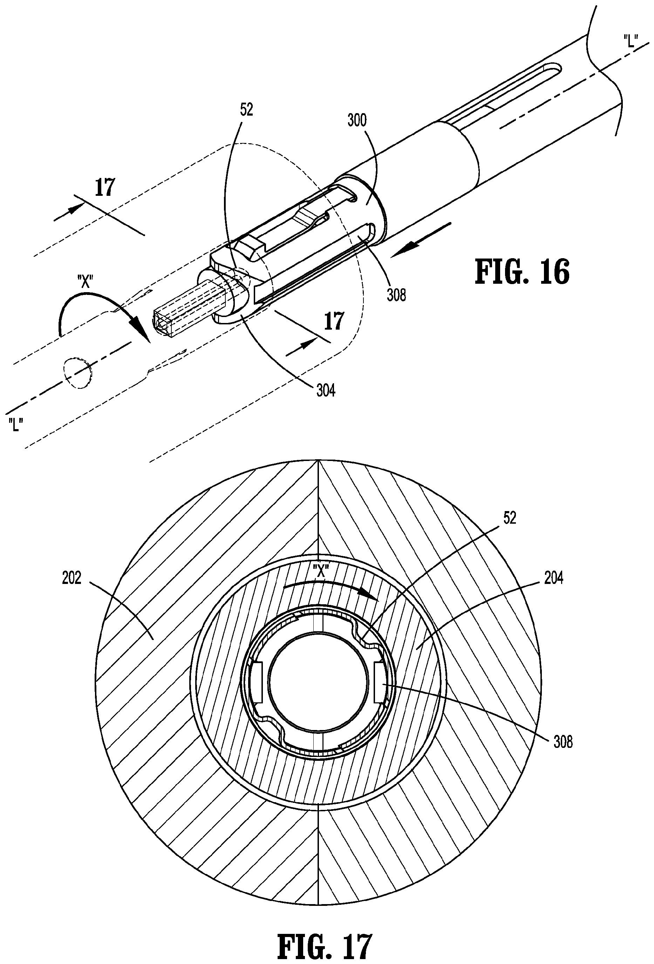

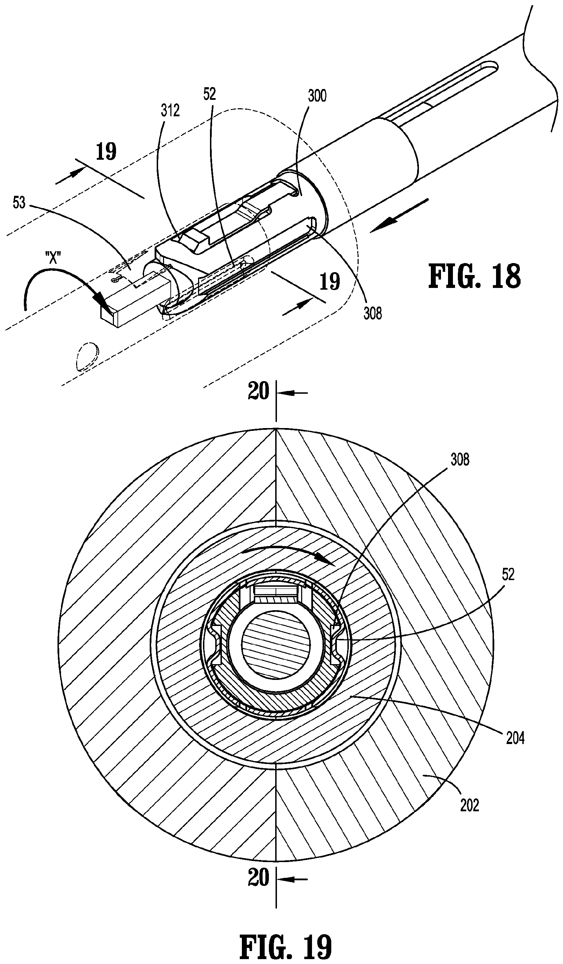

FIGS. 14 and 15 illustrate a method of loading the reloadable cartridge 50 onto the engagement portion 300 of the endoscopic tack retaining/advancing assembly 30. The reloadable cartridge 50 is secured with the inner body portion 204 and is rotatably supported within the outer body portion 202 of the shipping clip 200. FIGS. 16-19 illustrate coupling of the engagement portion 300 of the endoscopic tack retaining/advancing assembly 30 with the reloadable cartridge 50 by first inserting the mating tip 32a of the drive shaft 32 into the lumen 56 (FIG. 6) of the reloadable cartridge 50. As the engagement portion 300 is advanced farther into the lumen 56, the cartridge orientation guides 52 of the reloadable cartridge 50 engage the camming surface 304 of the engagement portion 300, which rotates the reloadable cartridge 50 in the direction of, e.g., an arrow "X", about the longitudinal axis "L-L" until the cartridge orientation guides 52 are in registration with the respective orientation slots 308 of the engagement portion 300. At this time, the cartridge orientation guides 52 may be slidably received into the respective orientation slots 308. FIG. 20 illustrates the cartridge locking portion 53 of the reloadable cartridge 50 that is biased radially inwards. As the cartridge orientation guides 52 slide into the respective orientation slots 308 of the engagement portion 300, the cartridge locking portion 53 of the reloadable cartridge 50 is received into the recess 312 defined by the engagement portion 300 and engages a support wall 310 of the engagement portion 300. Under such a configuration, when the cartridge locking portion 53 engages the support wall 310, the reloadable cartridge 50 is secured with the engagement portion 300.

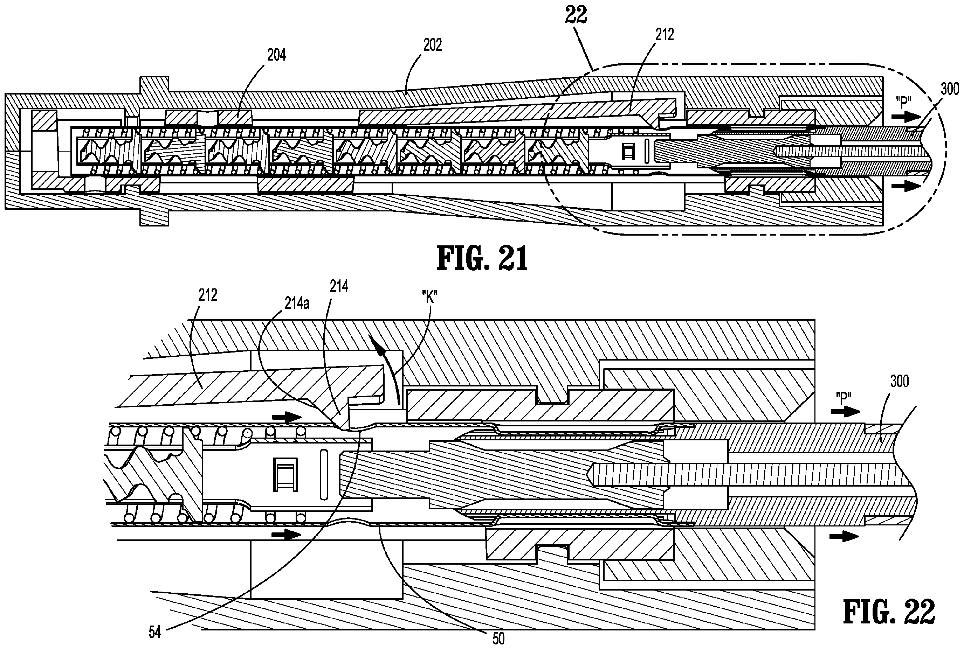

FIGS. 21 and 22 illustrate a method of separating the shipping clip 200 from the reloadable cartridge 50 which is now attached to the engagement portion 300 of the endoscopic tack retaining/advancing assembly 30. As the engagement portion 300 is pulled away from the shipping clip 200 in the direction of an arrow "P", the ramp 214a of the protrusion 214 slides out of the bore 54 of the reloadable cartridge 50, e.g., the finger 212 of the inner body portion 204 of the shipping clip 200 is pivoted in the direction of an arrow "K". In this manner, the shipping clip 200 is detached from the reloadable cartridge 50 now coupled to the endoscopic tack retaining/advancing assembly 30 (see FIG. 23). At this time, the endoscopic surgical tack applier 10 may be used as needed by the clinician to apply the tacks 80 to a desired surgical site. The button 22 on the handle assembly 20 may be pressed by the clinician to eject tacks 80 from the reloadable cartridge 50.

During or after the surgical procedure, the reloadable cartridge 50 may be removed from the endoscopic tack retaining/advancing assembly 30 or replaced with a new reloadable cartridge 50. The clinician may operate the slider 26 of the handle assembly 20 to mechanically or electro-mechanically retract the drive shaft 32 in the direction of an arrow "R". As the drive shaft 32 is retracted, the release member 306a of the finger 306 of the engagement portion 300 rides on the transition portion 33c and to the second portion 33b of the head portion 33 of the drive shaft 32. In this manner, the release member 306a urges the cartridge locking portion 53 of the reloadable cartridge 50 radially outwards of the support wall 310. The cartridge locking portion 53 of the reloadable cartridge 50 that is biased radially inwards is now positioned out of the recess 312 of the engagement portion 300 such that the reloadable cartridge 50 is detachable from the engagement portion 300. A new reloadable cartridge 30 may be loaded onto the engagement portion 300 of the endoscopic tack retaining/advancing assembly 30 through the steps discussed hereinabove. The cartridge alignment mechanism 100 described hereinabove facilitates loading and unloading of the reloadable cartridge 50 on the engagement portion 300 of the endoscopic tack retaining/advancing assembly 300. In particular, the loading of the reloadable cartridge 50 may be affected without aligning the orientation of the reload cartridge 50 and the engagement portion 300. Rather, the cartridge alignment mechanism 100 self-aligns the two components irrespective of the initial relative orientation of the two components. It is also envisioned that the cartridge alignment mechanism 100 may be configured for use in a robotic surgical system.

While the disclosure has been shown in the drawings, it is not intended that the disclosure be limited thereto, as it is intended that the disclosure be as broad in scope as the art will allow and that the specification be read likewise. Therefore, the above description should not be construed as limiting, but merely as exemplifications. Those skilled in the art will envision other modifications within the scope and spirit of the claims appended hereto.

The invention may be described by reference to the following numbered paragraphs:-
1. A surgical tack applier comprising:
   a reloadable cartridge supporting a plurality of tacks, the reloadable cartridge including a cartridge orientation guide having an axial protrusion extending radially inwards along a length of the reloadable cartridge;
   a shipping clip including an outer body and an inner body rotatably supported within the outer body, the reloadable cartridge detachably secured with the inner body for concomitant rotation with the inner body; and
   a tack retaining and advancing assembly configured to selectively release or fire the plurality of tacks, the tack retaining and advancing assembly including an engagement portion detachably supporting the reloadable cartridge, the engagement portion including:
      an orientation member defining an orientation slot slidably receiving the axial protrusion of the cartridge orientation guide of the reloadable cartridge; and
      a camming surface that is tapered along a length of the engagement portion such that when the cartridge orientation guide of the reloadable cartridge is pushed against the camming surface, the reloadable cartridge rotates to align the axial protrusion of the cartridge orientation guide with the orientation slot of the orientation member of the engagement portion.
2. The surgical tack applier according to paragraph 1, wherein the inner body includes a flexible finger having a protrusion, the flexible finger being radially deflectable.
3. The surgical tack applier according to paragraph 2, wherein the reloadable cartridge defines a bore dimensioned to receive the protrusion of the flexible finger of the inner body.
4. The surgical tack applier according to paragraph 3, wherein the protrusion has a tapered surface that slides out of the bore of the reloadable cartridge when the reloadable cartridge and the inner body are axially moved apart.
5. The surgical tack applier according to paragraph 1, wherein the tack retaining and advancing assembly includes a drive shaft.
6. The surgical tack applier according to paragraph 5, wherein the drive shaft is rotatable to selectively release or fire the plurality of tacks from the reloadable cartridge, and axially slidable between a first position and a second position.
7. A surgical tack applier comprising:
   a reloadable cartridge supporting a plurality of tacks, the reloadable cartridge including a cartridge orientation guide;
   a shipping clip including an outer body and an inner body rotatably supported within the outer body, the reloadable cartridge detachably secured with the inner body for concomitant rotation with the inner body; and
   a tack retaining and advancing assembly configured to selectively release or fire the plurality of tacks, the tack retaining and advancing assembly including an engagement portion detachably supporting the reloadable cartridge, the engagement portion including:
      an orientation member operatively engaging the cartridge orientation guide of the reloadable cartridge; and
      a camming surface that is tapered such that when the cartridge orientation guide of the reloadable cartridge is pushed against the camming surface, the reloadable cartridge rotates to align the cartridge orientation guide with the orientation member of the engagement portion.
8. The surgical tack applier according to paragraph 7, wherein the reloadable cartridge includes a cartridge locking portion that is biased towards a lumen defined by the reloadable cartridge.
9. The surgical tack applier according to paragraph 8, wherein the cartridge locking portion defines an acute angle with respect to a longitudinal axis defined by the reloadable cartridge.
10. The surgical tack applier according to paragraph 9, wherein the engagement portion defines a recess and includes a finger disposed within the recess, the finger selectively engaging the cartridge locking portion to selectively secure the reloadable cartridge with the engagement portion.
11. The surgical tack applier according to paragraph 10, wherein the cartridge locking portion of the reloadable cartridge is transitionable between a secured position, in which, the cartridge locking portion is disposed within the recess of the engagement portion, and a releasable position, in which, the cartridge locking portion is disposed radially outwards of the recess.
12. The surgical tack applier according to paragraph 11, wherein the tack retaining and advancing assembly includes a drive shaft, the drive shaft being rotatable to selectively release or fire the plurality of tacks from the reloadable cartridge, and axially slidable between a first position and a second position,
   wherein when the drive shaft is in the first position, the cartridge locking portion of the reloadable cartridge is disposed within the recess such that the reloadable cartridge is securely coupled to the engagement portion; and
   when the drive shaft is in the second position, the finger of the engagement portion urges the cartridge locking portion of the reloadable cartridge out of the recess such that the reloadable cartridge is releasable from the engagement portion.
13. A cartridge alignment mechanism for use with a surgical device, the cartridge alignment mechanism comprising:
   a cartridge supporting a plurality of anchors, the cartridge including a cartridge orientation guide;
   a shipping clip including an outer body and an inner body rotatably supported within the outer body, the cartridge removably coupled to the inner body for concomitant rotation with the inner body; and
   an engagement portion detachably supporting the cartridge, the engagement portion including:
      an orientation member slidably engaging the cartridge orientation guide of the cartridge; and
      a camming surface leading into the orientation member such that when the camming surface is pushed against the cartridge orientation guide of the reloadable cartridge, the cartridge orientation guide of the cartridge aligns with and engages the orientation member of the engagement portion.
14. The cartridge alignment mechanism according to paragraph 13, wherein the engagement portion further includes a finger having a release member that is radially movable.
15. The cartridge alignment mechanism according to paragraph 14, wherein the cartridge defines a lumen and includes a cartridge locking portion biased radially inwards.
16. The cartridge alignment mechanism according to paragraph 15, wherein the release member of the engagement portion selectively engages the cartridge locking portion of the cartridge to selectively secure the cartridge with the engagement portion.
17. The cartridge alignment mechanism according to paragraph 13, wherein the inner body of the shipping clip includes a finger having a protrusion, the finger being radially deflectable.
18. The cartridge alignment mechanism according to paragraph 17, wherein the cartridge defines a bore configured to receive the protrusion of the finger of the inner body to secure the cartridge with the inner body for concomitant rotation.
19. The surgical tack applier according to paragraph 13, the cartridge orientation guide of the cartridge is a protrusion extending along a length of the cartridge.
20. The surgical tack applier according to paragraph 19, wherein the orientation member of the engagement portion is a slot slidably receiving the protrusion of the cartridge.

## Claims

1. A surgical tack applier comprising:
a reloadable cartridge supporting a plurality of tacks, the reloadable cartridge including a cartridge orientation guide having an axial protrusion extending radially inwards along a length of the reloadable cartridge;
a shipping clip including an outer body and an inner body rotatably supported within the outer body, the reloadable cartridge detachably secured with the inner body for concomitant rotation with the inner body; and
a tack retaining and advancing assembly configured to selectively release or fire the plurality of tacks, the tack retaining and advancing assembly including an engagement portion detachably supporting the reloadable cartridge, the engagement portion including:
an orientation member defining an orientation slot slidably receiving the axial protrusion of the cartridge orientation guide of the reloadable cartridge; and
a camming surface that is tapered along a length of the engagement portion such that when the cartridge orientation guide of the reloadable cartridge is pushed against the camming surface, the reloadable cartridge rotates to align the axial protrusion of the cartridge orientation guide with the orientation slot of the orientation member of the engagement portion.

2. The surgical tack applier according to claim 1, wherein the inner body includes a flexible finger having a protrusion, the flexible finger being radially deflectable.

3. The surgical tack applier according to claim 2, wherein the reloadable cartridge defines a bore dimensioned to receive the protrusion of the flexible finger of the inner body; preferably wherein the protrusion has a tapered surface that slides out of the bore of the reloadable cartridge when the reloadable cartridge and the inner body are axially moved apart.

4. The surgical tack applier according to any preceding claim, wherein the tack retaining and advancing assembly includes a drive shaft; preferably wherein the drive shaft is rotatable to selectively release or fire the plurality of tacks from the reloadable cartridge, and axially slidable between a first position and a second position.

5. A surgical tack applier comprising:
a reloadable cartridge supporting a plurality of tacks, the reloadable cartridge including a cartridge orientation guide;
a shipping clip including an outer body and an inner body rotatably supported within the outer body, the reloadable cartridge detachably secured with the inner body for concomitant rotation with the inner body; and
a tack retaining and advancing assembly configured to selectively release or fire the plurality of tacks, the tack retaining and advancing assembly including an engagement portion detachably supporting the reloadable cartridge, the engagement portion including:
an orientation member operatively engaging the cartridge orientation guide of the reloadable cartridge; and
a camming surface that is tapered such that when the cartridge orientation guide of the reloadable cartridge is pushed against the camming surface, the reloadable cartridge rotates to align the cartridge orientation guide with the orientation member of the engagement portion.

6. The surgical tack applier according to claim 5, wherein the reloadable cartridge includes a cartridge locking portion that is biased towards a lumen defined by the reloadable cartridge; preferably wherein the cartridge locking portion defines an acute angle with respect to a longitudinal axis defined by the reloadable cartridge.

7. The surgical tack applier according to claim 6, wherein the engagement portion defines a recess and includes a finger disposed within the recess, the finger selectively engaging the cartridge locking portion to selectively secure the reloadable cartridge with the engagement portion; preferably wherein the cartridge locking portion of the reloadable cartridge is transitionable between a secured position, in which, the cartridge locking portion is disposed within the recess of the engagement portion, and a releasable position, in which, the cartridge locking portion is disposed radially outwards of the recess.

8. The surgical tack applier according to claim 7, wherein the tack retaining and advancing assembly includes a drive shaft, the drive shaft being rotatable to selectively release or fire the plurality of tacks from the reloadable cartridge, and axially slidable between a first position and a second position,
wherein when the drive shaft is in the first position, the cartridge locking portion of the reloadable cartridge is disposed within the recess such that the reloadable cartridge is securely coupled to the engagement portion; and
when the drive shaft is in the second position, the finger of the engagement portion urges the cartridge locking portion of the reloadable cartridge out of the recess such that the reloadable cartridge is releasable from the engagement portion.

9. A cartridge alignment mechanism for use with a surgical device, the cartridge alignment mechanism comprising:
a cartridge supporting a plurality of anchors, the cartridge including a cartridge orientation guide;
a shipping clip including an outer body and an inner body rotatably supported within the outer body, the cartridge removably coupled to the inner body for concomitant rotation with the inner body; and
an engagement portion detachably supporting the cartridge, the engagement portion including:
an orientation member slidably engaging the cartridge orientation guide of the cartridge; and
a camming surface leading into the orientation member such that when the camming surface is pushed against the cartridge orientation guide of the reloadable cartridge, the cartridge orientation guide of the cartridge aligns with and engages the orientation member of the engagement portion.

10. The cartridge alignment mechanism according to claim 9, wherein the engagement portion further includes a finger having a release member that is radially movable; preferably wherein the cartridge defines a lumen and includes a cartridge locking portion biased radially inwards.

11. The cartridge alignment mechanism according to claim 10, wherein the release member of the engagement portion selectively engages the cartridge locking portion of the cartridge to selectively secure the cartridge with the engagement portion; preferably wherein the inner body of the shipping clip includes a finger having a protrusion, the finger being radially deflectable.

12. The cartridge alignment mechanism according to claim 11, wherein the cartridge defines a bore configured to receive the protrusion of the finger of the inner body to secure the cartridge with the inner body for concomitant rotation.

13. The surgical tack applier according to any of claims 9 to 12, the cartridge orientation guide of the cartridge is a protrusion extending along a length of the cartridge; preferably wherein the orientation member of the engagement portion is a slot slidably receiving the protrusion of the cartridge.
